# EUROPEAN PATENT APPLICATION

(11) **EP 3 184 624 A1**
(43) Date of publication of application: **28.06.2017**
(21) Application number: 15202094.7
(22) Date of filing: 22.12.2015
(51) Int. Cl.: C12N 1/06, C12Q 1/68, C12N 13/00

(54) **FREQUENCY OPTIMIZED DEVICES AND METHODS FOR MICROFLUIDIC SONICATION**

(71) Applicant: Diagenode S.A., 4102 Seraing (BE)
(72) Inventor: DELADRIERE, Jean-Luc, 4102 Seraing (BE); JONEJA, Aric, 4102 Seraing (BE)
(74) Representative: Pronovem

(57) **Abstract**

A method of fragmenting nucleic acids, such as DNA or RNA, or a method of disrupting cells contained in a microfluidic chip (10) by means of ultrasound includes a tuning step for finding an optimal frequency of operation of an ultrasound transducer (20). The tuning includes a frequency sweep within a frequency band compatible with the ultrasound transducer and measuring amplitudes of sound during the frequency sweep. The sound is generated by cavitation within a material (30) comprising a liquid phase and which is coupled to the ultrasound transducer. Based on the measured amplitudes of sound, an actual resonant frequency can be determined. A microphone (50) is used for measuring the amplitudes of sound which is mounted at a distance from the ultrasound transducer (20) and the microfluidic chip (10).

## Description

The present invention is related to methods and devices for fragmenting nucleic acids, such as DNA, and disrupting cells using ultrasound. In particular, the present invention is related to methods and devices using microfluidic channels for subjecting the cells or the nucleic acids to ultrasound. More particularly, the present invention is related to finding an optimal frequency of sonication for the above kind of methods and devices.

There is an increasing interest in using small table top devices for fragmenting DNA in small sample volumes. In particular, microfluidic channels provided on a chip allow to effectively sonicate very small DNA samples in a cost effective manner with the additional advantage of making it possible to further process the sample on or within the chip. These processes are sometimes referred to as microfluidic sonication. To this end, it is known from "Fragmentation of DNA in a sub-microliter microfluidic sonication device", Q. Tseng et al., DOI: 10.1039/c2lc40595d to use a PDMS chip for sonicating 7 µL of DNA with a Langevin-type composite ultrasound transducer operable at 63 kHz. The transducer and the PDMS chip were bonded on a glass substrate using a thermo-reversible epoxy glue, and such that there was no direct contact between the transducer and the chip. The acoustic energy from the transducer is transmitted to the chip through a flexural vibration mode of the glass plate and resulted in a shifted resonance frequency of 74.3 kHz.

A drawback of the above system, is that testing of different samples in different microfluidic chips with a same ultrasound transducer is cumbersome. An exact positioning between transducer and chip is required, in order to have optimal vibration coupling through the flexural mode. In order to treat a different sample, one would need to heat the thermo-reversible epoxy glue in order to remove the chip from the transducer, clean the transducer to remove any residue, and carefully position and glue a new chip on the transducer.

From "LCAT DNA Shearing", Y. Okabe and A. P. Lee, DOI: 10.1177/2211068213495546, it is known to fragment DNA using lateral cavity acoustic transducers. This is a microfluidic chip in which lateral air pockets (lateral cavities) in fluid communication with the microfluidic channel are provided. These lateral cavities are believed to introduce controlled microstreaming into the channel, which allows for a sharper peak of DNA fragments. To ensure a minimal loss of signal transmission between the ultrasound transducer and the microfluidic chip, the chip is placed over the transducer and coupled thereto using ultrasound gel. By using ultrasound gel as coupling medium and placing the microfluidic chip on top of the piezoelectric transducer, the fragmenting of different samples becomes easier.

However, a drawback is that different amounts of ultrasound gel used each time and different microfluidic chips with different geometry and/or mass used shifts the frequency at which resonance occurs, resulting in a less than optimal sonication. Yet another problem is that the ultrasound transducers have an inherent resonant frequency which varies from part to part within a given model number. Furthermore, the mechanical coupling/fixture between the transducer and a(n external) support, such as a tabletop, introduce a further frequency shift which is difficult to replicate between different parts and which may further vary with time. It has been observed that the resonant frequency of the whole system may vary by several kHz.

US 2007/0114306 (Kawakami et al.) 24.05.2007 describes an apparatus for micro particle production including a treatment chamber for the liquid to be treated, a laser light source for micro particulation, and an ultrasonic transducer. The document describes that when the ultrasonic transducer is attached to a side wall of the treatment chamber, a microphone can be mounted at an opposite side to monitor a vibration amplitude of the treatment chamber. However, when the ultrasonic transducer is installed at the bottom of the treatment chamber, the document describes that the microphone is not required since the voltage applied to the transducer would automatically increase when the resonant vibration state of the treatment chamber is attained.

A difference between US 2007/0114306 and micro sonication as used in the present invention, is that the amounts of liquid that are treated are much smaller. Whereas liquid samples on the order of 3 ml are used in the former, samples on the order of a few µl are used with micro sonication, which renders monitoring the treated sample more difficult.

US 2009/0052272 (Sarvazyan) 26.02.2009 describes ultrasound assisted contactless stirring of liquids in a resonator cell by repeated creating and destruction of nodal patterns associated with standing waves of various resonance frequencies. A swept frequency mode of sonication is used requiring a broadband source of ultrasound and requiring low levels of ultrasound intensity which are smaller than those needed to produce cavitational effects. This requires a specific type of transducers, as the common piezoelectric ultrasound transducers have a narrow operating frequency band, and is not cost-effective.

An objective of aspects of the present invention is to provide methods and devices for microfluidic sonication which have improved performance. It is an object of aspects of the invention to increase the throughput of microfluidic sonication devices and methods by reducing sonication time.

According to a first aspect of the invention, there is therefore provided a method of fragmenting nucleic acids, such as DNA or RNA, or a method of disrupting cells as set out in the appended claims. The method includes a tuning step for finding an optimal frequency of operation of the ultrasound transducer, having regard to the system as installed, i.e. including the ultrasound transducer with its mechanical clamping or support, and optionally including the microfluidic chip and any coupling between the microfluidic chip and the ultrasound transducer. The tuning includes a frequency sweep within a frequency band compatible with the ultrasound transducer and measuring amplitudes of sound during the frequency sweep. The sound is generated by cavitation within a material comprising a liquid phase and which is coupled to the ultrasound transducer. Based on the measured amplitudes of sound, an actual resonant frequency can be determined. Advantageously, a microphone is used for measuring the amplitudes of sound which is mounted at a distance from the ultrasound transducer and the microfluidic chip.

The material in which cavitation occurs during the frequency sweep can be present within the microfluidic chip, or outside the chip. In an advantageous aspect, it has been observed that interposing a gel, such as an ultrasound gel, between the microfluidic chip and the ultrasound transducer, allows to create cavitation in the gel during sonication. As the amount of gel commonly used is much larger than the contents of the microfluidic container, a strong cavitation noise due to cavitation in the gel is observed, which allows a reliable capturing of sound and improved determination of the optimal frequency. Furthermore, the amount of cavitation in the gel will be a direct indication of resonance of the transducer. When the ultrasound transducer is operated at the frequency corresponding to a maximum of the sound amplitude, an optimal shearing protocol can be obtained, which will deliver a maximum of energy to the sample contained in the microfluidic chip.

According to a second aspect of the invention, there is provided a kit of parts as set out in the appended claims. The kit according to the second aspect of the invention advantageously allows to carry out method steps according to the first aspect of the invention.

Aspects of the invention will now be described in more detail with reference to the appended drawings, wherein same reference numerals illustrate same features and wherein:
Figure 1 represents a setup for microfluidic sonication according to aspects of the present invention, including a microfluidic container and an ultrasound generating device;
Figure 2 represents a top view of the microfluidic container of Fig. 1, including a microfluidic channel, and mounted on the bearing surface of an ultrasonic transducer;
Figure 3 represents a top view of another microfluidic container including a microfluidic chamber which can be used in the present invention, shown mounted on the bearing surface of an ultrasonic transducer;
Figure 4 represents an electrical circuit for driving the microphone according to an aspect of the present invention.

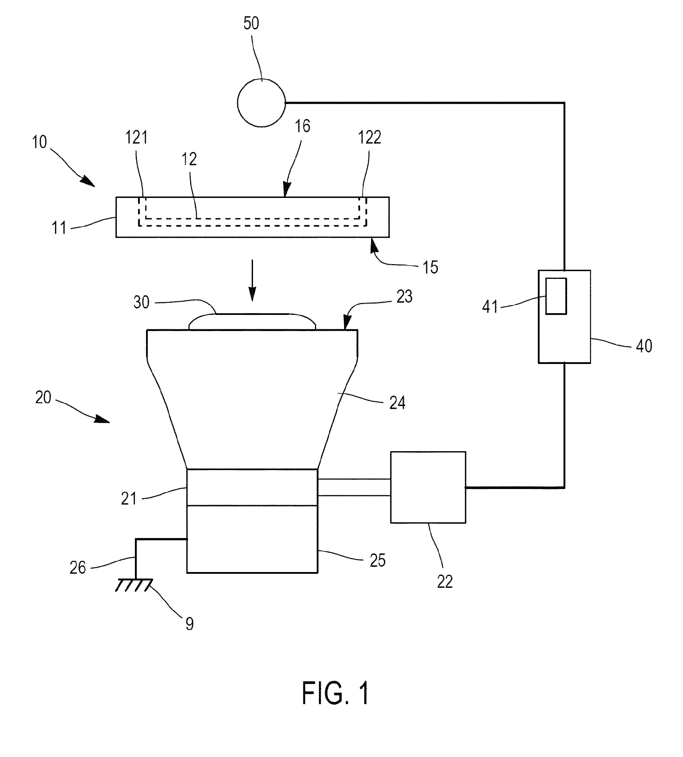

Referring to Fig. 1, a microfluidic container 10 or chip comprises a housing 11, having an advantageously flat (planar) bottom surface 15, and an optionally flat top surface 16. The housing can be made, fully or in part, of an advantageously transparent material, such as glass or a polymeric material. The housing can be composed of different, layered materials, as described further below.

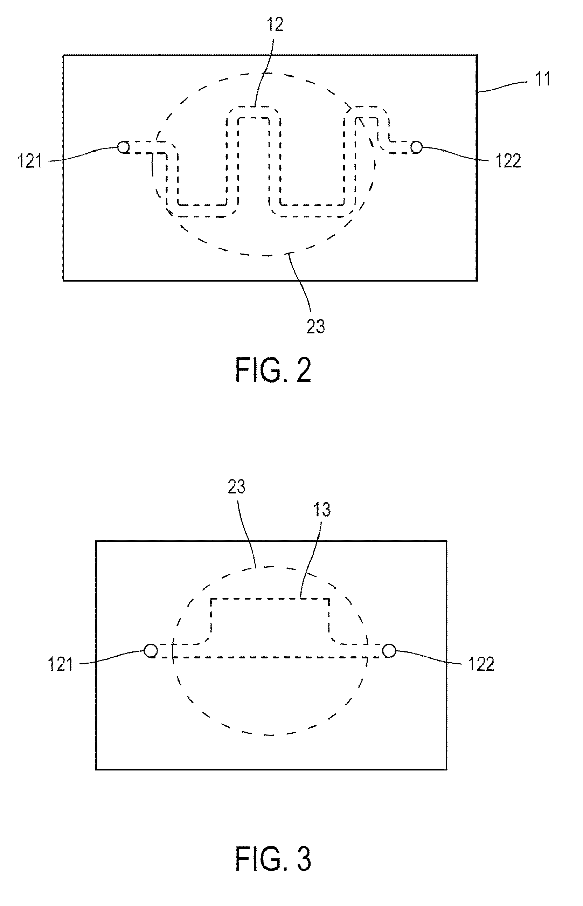

A microfluidic chamber 12 is provided within the housing 11, generally interposed between the top surface 16 and the bottom surface 15. Generally, the microfluidic chamber will comprise an inlet 121 for injecting a sample, such as a DNA sample, to be sonicated into the chamber, and an outlet 122 for evacuating the sample. Additional inlets and/or outlets may be provided as desired. There is no limitation on the shape of the microfluidic chamber 12, which can have any suitable shape. Possible shapes are represented in Fig. 2, showing the microfluidic chamber shaped as a serpentine-like channel 12 and in Fig. 3, showing the microfluidic chamber shaped as a bulk chamber 13. Other suitable arrangements, such as a network of channels of same diameter or different diameters, are known to, or can be easily thought of by the skilled person.

It will be convenient to note that the term chamber as used in the present description does include channel or any other suitable network of liquid passageways.

Advantageously, the microfluidic chamber 12, 13 is formed as a closed chamber or channel, i.e. it has closed cross sectional perimeters, as opposed to open channels which generally have only three sides and are open to the top.

The term "microfluidic" in microfluidic chamber refers to such chambers having a liquid containing capacity of 500 µl or less, advantageously 250 µl or less, advantageously 150 µl or less, advantageously 100 µl or less, advantageously 80 µl or less, advantageously 65 µl or less, advantageously 50 µl or less, advantageously 30 µl or less, advantageously 25 µl or less, advantageously 20 µl or less, advantageously 15 µl or less. Microfluidic chambers as used in aspects of the present invention can have a volume of at least at least 1 nl, optionally at least 10 nl, optionally at least 100 nl, optionally at least 1 µl.

The microfluidic chamber 12, 13 generally will have a rectangular cross-section (as considered in a vertical plane), even though other shapes can be contemplated. The microfluidic chamber can have an (equivalent) diameter smaller than or equal to 5 mm, advantageously smaller than or equal to 3 mm, advantageously smaller than or equal to 2.5 mm. The diameter is preferably measured in a vertical plane. For a rectangular cross-section, the channel 12 or chamber 13 can have a width smaller than or equal to 5 mm, such as between 3 mm and 500 µm, or less. The channel 12 or chamber 13 can have a height smaller than or equal to 1 mm, advantageously smaller than or equal to 500 µm, such as between 300 µm and 75 µm, or less.

The above microfluidic chambers can be manufactured in a plurality of ways. A first possibility is to machine as a pocket in a piece of polymeric material, and closing the pocket by covering the piece with a sheet of a same or different material (e.g. glass covering). The piece of polymeric material and the covering together will constitute the housing 11 of the microfluidic container 10. A second possibility is to injection-molding a base of the housing of the microfluidic container, including the channels/chambers, and then seal by ultrasonically welding a cap or cover to seal the channels/chambers. It was demonstrated that this works with materials such as polystyrene, polycarbonate, poly(methyl methacrylate) (PMMA), styrene methyl methacrylate (SMMA), methyl methacrylate-butadiene-styrene plastic (MBS) and possibly modified styrene acrylic copolymers, such as Zylar®. Still alternatively, the housing can be made by bonding several stacked layers, where the middle layer is comprised of a double-sided tape. The channels/chambers are advantageously cut out in the tape using a laser or die cutter, and are sealed on the top and bottom using layers of polymer or glass. It was established that the polymer can be PMMA, polycarbonate, polyester, or polystyrene. It was established that the tape material can be polypropylene or polyester, and the adhesive on each side can be silicone- or acrylic- based. The top, bottom, or both the top and bottom of the housing 11 advantageously are flat (planar).

The microfluidic container 10 is subjected to the action of ultrasound waves by coupling it to an ultrasonic transducer 20. Ultrasonic transducer 20 is advantageously a piezoelectric transducer, although other types, such as magnetostrictive may alternatively be used. Piezoelectric transducers comprise a piezoelectric array 21 as actuating component interposed between a mount 25 and a seismic mass 24. Due to the small dimensions of the transducer 20, it will generally be required to fix the mount 25. To this end, transducer 20 may be assembled to a fixture, such as a mechanical clamp 26 for fixing to e.g. to a tabletop 9. Piezoelectric array 21 is operable to vibrate the seismic mass 24 coupled to it. A driver 22 can be electrically coupled to the piezoelectric array 21 for supplying it with electrical power at a predetermined frequency such that the seismic mass 24 vibrates at or nearby a resonant frequency.

Suitable ultrasonic transducers advantageously operate at a frequency between 20 kHz and 100 kHz, advantageously 80 kHz or less, advantageously at least 30 kHz. A suitable ultrasonic transducer is sold by Hainertec (China), model HNC-4AH-2560 which is a transducer operating at about 60 kHz.

The ultrasound transducer 20 comprises a top surface 23, which is a top surface of the seismic mass 24 and which is arranged for receiving the microfluidic container 10. Coupling means can be provided for coupling the microfluidic container 10 to the surface 23. The coupling means can be in the form of a coupling medium. A gel 30, such as any suitable ultrasound gel, can be used as a coupling medium, to ensure proper energy transmission between the vibrating seismic mass 24 and the microfluidic container 10. suitable gels are described in co-pending Belgian patent application No. BE2014/0742 (DIAGENODE) filed on 29.09.2014, the contents of which are incorporated herein by reference. The microfluidic container 10 can be placed on surface 23 such that it is aligned to provide at least a partial overlap between the surface 23 and the channel 12 or chamber 13 when vertically projected as indicated in figures 2 and 3.

Additional coupling means can be used in addition to the gel, in order to couple the microfluidic container 10 to the surface 23/seismic mass 24 and hence to ultrasonic transducer 20, such as mechanical coupling means, e.g. a snap fit connector or set screw.

A control unit 40 is operably connected to driver 22. Control unit 40 is advantageously arranged to set a frequency and/or a power level for the driver 22, in order to operate ultrasonic transducer 20. Advantageously control unit 40 is arranged to set a multitude of frequencies and/or power levels. Advantageously, the driver is configured for operating at a multitude of frequencies, possibly within a predetermined frequency band.

Control unit 40 is further operably connected to a microphone 50. Microphone 50 is advantageously mounted to capture sound emanating from the assembly ultrasonic transducer 20/microfluidic container 10. The microphone 50 may be mounted at a distance from ultrasonic transducer 23, possibly at a distance from surface 23. Microphone 50 is advantageously mounted at a distance from microfluidic container 10 when assembled on surface 23. Microphone 50 is advantageously mounted above the transducer 20 (above surface 23) and advantageously above the microfluidic container 10 as mounted/placed on the surface 23. It was observed that placing the microphone 50 directly above the microfluidic container gave an optimal signal to noise ratio.

Cavitation occurs in the liquid (e.g. sample DNA solution) contained in the microfluidic chamber 12, 13 of container 10 when the ultrasonic transducer 20 is operated at the above indicated frequencies and the container is coupled to the transducer. Without wishing to be bound by theory, it is believed that cavitation is the mechanism through which DNA is fragmented/sheared. It has furthermore been observed that cavitation also occurs in the gel 30 in such cases. Cavitation may emanate a sound, generally referred to as white noise, whose intensity level is maximum at the frequency of resonance of the ultrasonic transducer. The resonance frequency is influenced by the manner of fixing mount 25, e.g. through fixture 26, and any support used for fixing transducer 20/mount 25 to (e.g. the tabletop 9). These latter features may differ between end users, and therefore the actual resonance frequency of transducer 20 may differ between end users, and possibly even between different test runs.

In aspects of the invention, the white noise intensity level, due to cavitation, is measured for different frequencies in order to find the actual resonance frequency of the transducer assembly. Microphone 50 is operable to capture the sound of cavitation of the liquid in microfluidic chamber 12, 13 and/or of the gel 30 and convert it to an electrical signal which is processed by control unit 40. According to an aspect of the invention, control unit 40 is configured to perform an automatic tuning of the assembly in order to find the actual resonance frequency. Control unit 40 is advantageously configured to determine a peak amplitude of the sound captured by microphone 50. Advantageously, control unit 40 is configured to consecutively set a plurality of different frequencies to driver 22 at which transducer 20 will shortly operate. This is referred to as frequency sweep. Simultaneously, control unit 40 is configured to determine a peak intensity level (peak amplitude) of the sound captured by microphone 50 for the plurality of frequencies set. The plurality of different frequencies are advantageously selected within a predetermined frequency band. By way of example, the control unit 40 can be implemented to increase (or decrease) a frequency set to driver 22 (i.e. a frequency of the power signal applied to piezoelectric array 21) in steps of between 5 Hz to 100 Hz. This stepwise frequency change advantageously occurs within a narrow frequency band, such as a frequency band of 15 kHz or less, e.g. 10 kHz or less, selected around a starting frequency for the tuning procedure, e.g. 60 kHz.

Control unit 40 is advantageously configured to set a frequency of operation of transducer 20 (for fragmenting the nucleic acids) corresponding to the peak amplitude of sound as determined through the frequency sweep.

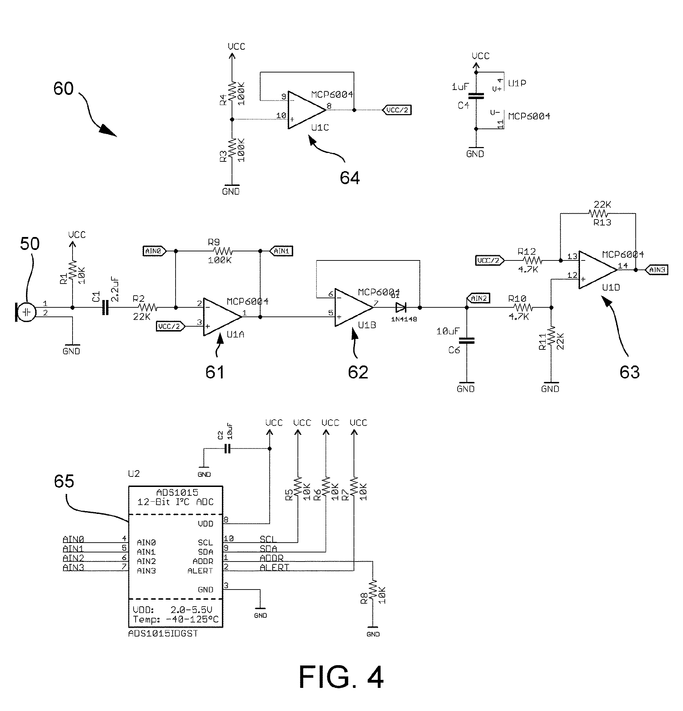

Control unit 40 advantageously comprises signal processing means, which may be in the form of an electric circuit. Figure 4 shows one possible electric circuit 60 suitable for processing signals from microphone 50. Circuit 60 comprises an analog signal conditioning part, generally configured for amplifying a signal from microphone 50. The signal may pass a first operational amplifier 61 configured as an inverting amplifier, e.g. with a gain of between 2 and 6, e.g. 3 or 4.5. Circuit 60 can comprise a second operational amplifier 62, configured to hold a maximum value of the analog signal. A third operational amplifier 63 can be operable to remove a voltage bias, e.g. a 2.5 V bias. To this end, a fourth operational amplifier 64 is provided, configured to provide a virtual ground. Third operational amplifier 63 can be configured to amplify the signal to have it in a desired voltage range, e.g. between 0 and 5 V.

Circuit 60 can comprise a digital signal processing part, generally comprising an analog to digital converter (ADC) 65. ADC 65 is configured to transform the amplitude of the signal applied to it into a digital signal, e.g. a 12 bit signal. Control unit 40 advantageously comprises a microcontroller 41 operably connected to ADC 65. Microcontroller 41 can be implemented with logic, e.g. a computer-implemented program, for further processing the digital signal. By way of example, the microcontroller may compare the digital signal with other readings, and may store the digital signal in memory, together with a corresponding frequency set to driver 22. Microcontroller 41 is advantageously implemented to store the frequency corresponding to a maximal amplitude of sound and to set this frequency in control unit 40 as an operating frequency for further operation of the transducer, e.g. for nucleic acid fragmenting/shearing procedures. By way of example, control unit 40 may be implemented with a shearing protocol, including a time period for operating transducer 20. The frequency determined and set above may then be used as operating frequency for this shearing protocol.

Control unit 40, through e.g. microcontroller 41, may be configured to adapt a sonication power level in order to avoid saturating the microphone 50 prior to performing the frequency sweep as described above. To this end, control unit 40 can be configured to set a determined power level, e.g. a maximum power level, in addition to a frequency, to driver 22 for transmission to the piezoelectric array 21. The microphone 50 and/or signal processing circuit 60 may become saturated by the sound captured when transducer 20 is operated at the determined power level. Control unit 40 can then be configured to stepwise reduce (or, in the inverse case, increase) the power level until a desired (e.g., non-saturating) sound level is captured by microphone 50.

As the cavitation noise in the ultrasound gel will generally be of higher intensity than the cavitation noise generated by the liquid sample in the microfluidic chamber, it may be easier for the microphone to detect a maximal sound amplitude when using ultrasound gel. It will however be convenient to note that methods of the invention may well be carried out without the use of ultrasound gel, e.g. where the microfluidic container 10 is coupled to the ultrasound transducer 20 by other means than gel, e.g. mechanically through a snap-fit connector, screw, or otherwise. In the latter case, the microphone 50 may record/capture the cavitation noise emanating from a liquid sample contained in the microfluidic container 10, and the optimal (resonant) frequency is advantageously determined following same procedures as described above.

Even though aspects of the invention have been described using the terms 'liquid' and 'gel', it will be convenient to note that any other suitable material capable of forming cavitation when excited at the indicated frequencies can be used for determining the optimal/resonant frequency of the assembly, e.g. materials containing a liquid phase, such as (hydro)colloids, emulsions, sols.

It will be convenient to note that the frequency tuning as described above may be a procedure to be applied once, when the transducer 20 is installed at the end user. The tuning procedure can be performed by coupling a microfluidic container 10 or dummy to the transducer 20. In case ultrasound gel is used as coupling medium between the surface 23 and the bottom surface 15 of the container 10 (or dummy), the microfluidic container may be empty (e.g. void of any liquid). The control unit will then perform a frequency sweep and collect sound intensity levels through microphone 50 to determine a maximum sound level and corresponding frequency to set as operating frequency. Alternatively, the frequency tuning procedure can be applied each time prior to performing a batch of test runs. It will be convenient to note that it is possible to perform a frequency tuning as described above without using a container or dummy, e.g. by applying only gel on the transducer surface 23. In the latter case however, it has been observed that the cavitation in the gel produces a weaker sound level.

It will be convenient to note that the mass of the microfluidic container 10 is generally insignificant compared to seismic mass 24. The resonant frequency will therefore not be significantly affected by using slightly different microfluidic containers or empty containers for the frequency sweep (in case of using ultrasound gel).

## Claims

1. Method of fragmenting nucleic acids, such as DNA or RNA, or disrupting cells, comprising:
coupling a microfluidic container (10) having a liquid containing capacity of 500 µL or less to a surface (23), and
vibrating the surface (23) by operating an ultrasound transducer (20) at a frequency of operation to fragment nucleic acids or disrupt cells contained in solution in the microfluidic container (10), **characterised in that** prior to the step of vibrating the surface (23), the method comprises:
coupling a material (30) capable of forming cavitation within the material to the surface (23),
vibrating the surface (23) by operating the ultrasound transducer (20) sequentially at a plurality of different frequencies, wherein cavitation occurs in the material, the cavitation generating a sound,
measuring a plurality of amplitudes of the sound corresponding to the plurality of different frequencies,
selecting one of the plurality of different frequencies as the frequency of operation based on the measured plurality of amplitudes of the sound.

2. Method of claim 1, wherein the material is a gel (30) applied on the surface (23).

3. Method of claim 2, wherein the gel (30) forms a coupling medium between the microfluidic container (10) and the surface (23).

4. Method of any one of the preceding claims, wherein the material comprises a liquid phase, the material being contained in a liquid passageway (12, 13) of the microfluidic container (10), wherein the microfluidic container is coupled to the surface (23).

5. Method of any one of the preceding claims, wherein the plurality of different frequencies are comprised between 20 kHz and 80 kHz.

6. Method of any one of the preceding claims, wherein the plurality of different frequencies fall within a band of 15 kHz or less.

7. Method of any one of the preceding claims, wherein the sound generated by the cavitation is white noise.

8. Method of any one of the preceding claims, wherein the frequency of operation is selected as the one of the plurality of different frequencies corresponding to a maximum of the plurality of amplitudes of the sound.

9. Method of any one of the preceding claims, wherein the plurality of amplitudes of the sound are measured by a microphone spaced apart from the surface and from the microfluidic container.

10. Method of any one of the preceding claims, wherein prior to operating the ultrasound transducer at the plurality of different frequencies, the method comprises:
operating the ultrasound transducer sequentially at a plurality of different power levels, wherein the ultrasound transducer is operated at a predetermined frequency,
measuring a plurality of second amplitudes of the sound corresponding to the plurality of different power levels and the predetermined frequency, and
selecting one of the plurality of different power levels based on the plurality of second amplitudes of the sound, wherein the ultrasound transducer is operated at the selected one of the plurality of different power levels when operating the ultrasound transducer at the plurality of different frequencies.

11. Kit, comprising:
an assembly comprising:
an ultrasound transducer (20) in operable connection to a surface (23),
a support (25, 26, 9) for the ultrasound transducer, and
a control unit (40) operable to operate the ultrasound transducer (20) at a frequency of operation,
a microfluidic container (10) having a liquid containing capacity of 500 µL or less,
**characterised in that** the assembly comprises a microphone (50) operably connected to the control unit (40),
and **in that** the control unit (40) is operable to execute the method steps of any one of the preceding claims.

12. Kit of claim 11, wherein the microphone (50) is spaced apart from the surface (23) and in operation is spaced apart from the microfluidic container (10).

13. Kit of claim 11 or 12, wherein the microfluidic container (10) comprises:
a housing (11) extending between a lower surface (15) and an upper surface (16),
a microfluidic chamber (12, 13) provided in the housing, wherein the microfluidic chamber has a closed cross sectional perimeter and has a liquid containing capacity equal to or smaller than 500 µl.

14. Kit of claim 13, wherein the microfluidic chamber (12, 13) comprises a microfluidic channel having a closed cross sectional perimeter and an equivalent diameter smaller than or equal to 5 mm.

15. Kit of any one of the claims 11 to 14, comprising a gel (30) suitable for forming a coupling medium between the microfluidic container (10) and the surface (23) by interposition between the microfluidic container and the surface.
